(19) Europäisches Patentamt
European Patent Office
Office européen des brevets

(11) **EP 1 645 300 B1**

(12) **EUROPEAN PATENT SPECIFICATION**

(45) Date of publication and mention
of the grant of the patent:
**13.08.2008 Bulletin 2008/33**

(51) Int Cl.:
*A61L 31/14* *(2006.01)*          *A61L 31/04* *(2006.01)*
*A61F 2/06* *(2006.01)*

(21) Application number: **05256006.7**

(22) Date of filing: **27.09.2005**

(54) **Improved geometry and non-metallic material for controlled recoil stent**

Verbessertes geometrisches und nicht-metallisches Material für einen Stent mit Kontrolliertem Rückschlag

Géométrie améliorée et matériau non métallique pour le recul contrôlé des stents

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.10.2004 US 961127**

(43) Date of publication of application:
**12.04.2006 Bulletin 2006/15**

(73) Proprietor: **Cordis Corporation**
**Miami Lakes,**
**Florida 33014 (US)**

(72) Inventors:
• **Burgermeister, Robert**
**Bridgewater, NJ 08807 (US)**
• **Dave, Vipul**
**Hillsborough, NJ 08844 (US)**
• **Grishaber, Randy-David Burce**
**Asbury, NJ 08802 (US)**

(74) Representative: **Belcher, Simon James**
**Urquhart-Dykes & Lord LLP**
**Tower North Central**
**Merrion Way**
**Leeds LS2 8PA (GB)**

(56) References cited:
US-A1- 2002 058 989          US-A1- 2003 083 732
US-A1- 2004 059 408          US-B1- 6 334 870
US-B1- 6 395 020

**Description**

The present invention relates to novel geometries for use in implantable medical devices, and more particularly, to novel stent designs manufactured or fabricated from alloys that provide high strength, high flexibility, high expansion capability, high fatigue resistance and controlled recoil. The present invention also relates to biocompatible non-metallic materials that provide for designed in microstructures that facilitate the design of devices with a wide range of geometries that are adaptable to various loading conditions.

Currently manufactured intravascular stents do not adequately provide sufficient tailoring of the microstructural properties of the material forming the stent to the desired mechanical behavior of the device under clinically relevant in-vivo loading conditions. Any intravascular device should preferably exhibit certain characteristics, including maintaining vessel patency through a chronic outward force that will help to remodel the vessel to its intended luminal diameter, preventing excessive radial recoil upon deployment, exhibiting sufficient fatigue resistance and exhibiting sufficient ductility so as to provide adequate coverage over the full range of intended expansion diameters.

US-2003-A-0083732 discusses processes for forming stents from polymers.

Accordingly, there is a need to develop precursory materials and the associated processes for manufacturing intravascular stents that provide device designers with the opportunity to engineer the device to specific applications.

The present invention overcomes the limitations of applying conventionally available materials to specific intravascular therapeutic applications as briefly described above.

According to the present invention, there is provided an intraluminal scaffold as defined in appended claim 1. The intraluminal scaffold comprises at least one load bearing element having a luminal surface and an abluminal surface, the load bearing element having a predetermined wall thickness, wherein the wall thickness is defined by the radial distance between the luminal surface and the abluminal surface, and a predetermined feature width, wherein an area bounded by the wall thickness and the feature width comprises three zones, a first zone, a second zone, and a neutral zone, which are the result of a stress gradient that is directly proportional to external loading conditions, and wherein when there is a change in external loading conditions either from a loaded or an unloaded condition the first zone undergoes a change in compressive and/or tensile stress due to the change in external load, the second zone undergoes a change in tensile and/or compressive stress due to the change in external load and the neutral zone is substantially stress free and is between the first and second zones, the feature width being the linear distance across the first, neutral and second zones in a direction substantially orthogonal to the wall thickness, the load bearing element being fabricated from a non-metallic material processed to have a microstructure with structural domains having a size of 50 microns or less and at least one internal structural boundary within the bounded area.

The biocompatible material for implantable medical devices of the present invention offers a number of advantages over currently utilized materials. The biocompatible material of the present invention is magnetic resonance imaging compatible, is less brittle than other metallic materials, has enhanced ductility and toughness, and has increased durability. The biocompatible material also maintains the desired or beneficial characteristics of currently available metallic materials, including strength and flexibility.

The biocompatible material for implantable medical devices of the present invention may be utilized for any number of medical applications, including vessel patency devices such as vascular stents, biliary stents, ureter stents, vessel occlusion devices such as atrial septal and ventricular septal occluders, patent foramen ovale occluders and orthopedic devices such as fixation devices.

The biocompatible material of the present invention is simple and inexpensive to manufacture. The biocompatible material may be formed into any number of structures or devices.

The biocompatible material of the present invention comprises a unique composition and designed-in properties that enable the fabrication of stents that are able to withstand a broader range of loading conditions than currently available stents. More particularly, the microstructure designed into the biocompatible material facilitates the design of stents with a wide range of geometries that are adaptable to various loading conditions.

The biocompatible materials of the present invention are non-metallic materials, including polymeric materials. These non-metallic materials may be designed to exhibit properties substantially similar to the metallic materials described herein, particularly with respect to the microstructure design, including the presence of at least one internal grain boundary or its non-metallic equivalent; namely, spherulitic boundary.

Embodiments of the invention will now be described by way of example with reference to the accompanying drawings, in which:

Figure 1 is a planar representation of an exemplary stent fabricated from the biocompatible alloy useful for understanding the present invention;

Figure 2 is a detailed planar representation of a hoop of a stent fabricated from the biocompatible alloy useful for understanding the present invention; and

Figure 3 is a simplified schematic cross-sectional representation of an intraluminal scaffold element in accordance with the present invention.

Metallic alloys will now be discussed as an example, to help understand the present invention.

Biocompatible, solid-solution strengthened alloys such as iron-based alloys, cobalt-based alloys and titanium-based alloys as well as refractory metals and refractory-based alloys may be utilized in the manufacture of any number of implantable medical devices. The biocompatible alloy for implantable medical devices in accordance with the present invention offers a number of advantages over currently utilized medical grade alloys. The advantages include the ability to engineer the underlying microstructure in order to sufficiently perform as intended by the designer without the limitations of currently utilized materials and manufacturing methodologies.

For reference, a traditional stainless steel alloy such as 316L (i.e. UNS S31603) which is broadly utilized as an implantable, biocompatible device material may comprise Chromium (Cr) in the range from about 16 to 18 wt.%, nickel (Ni) in the range from about 10 to 14 wt.%, molybdenum (Mo) in the range from about 2 to 3 wt.%, manganese (Mn) in the range up to 2 wt.%, silicon (Si) in the range up to 1 wt.%, with iron (Fe) comprising the balance (approximately 65 wt.%) of the composition.

Additionally, a traditional Cobalt-based alloy such as L605 (i.e. UNS R30605) which is also broadly utilized as an implantable, biocompatible device material may comprise Chromium (Cr) in the range from about 19 to 21 wt.%, tungsten (W) in the range from about 14 to 16 wt.%, nickel (Ni) in the range from about 9 to 11 wt.%, iron (Fe) in the range up to 3 wt.%, manganese (Mn) in the range up to 2 wt.%, silicon (Si) in the range up to 1 wt.%, with Cobalt (cobalt) comprising the balance (approximately 49 wt.%) of the composition.

Alternately, another traditional Cobalt-based alloy such as Haynes 188 (i.e. UNS R30188) which is also broadly utilized as an implantable, biocompatible device material may comprise nickel (Ni) in the range from about 20 to 24 wt.%, chromium (Cr) in the range from about 21 to 23 wt.%, tungsten (W) in the range from about 13 to 15 wt.%, iron (Fe) in the range up to 3 wt.%, manganese (Mn) in the range up to 1.25 wt.%, silicon (Si) in the range from about 0.2 to 0.5 wt.%, lanthanum (La) in the range from about 0.02 to 0.12 wt.%, boron (B) in the range up to 0.015 wt.% with cobalt (Co) comprising the balance (approximately 38 wt.%) of the composition.

In general, elemental additions such as chromium (Cr), nickel (Ni), tungsten (W), manganese (Mn), silicon (Si) and molybdenum (Mo) were added to iron- and/or Cobalt-based alloys, where appropriate, to increase or enable desirable performance attributes, including strength, machinability and corrosion resistance within clinically relevant usage conditions.

Preferred material characteristics of a stent include strength, fatigue robustness and sufficient ductility.

Strength is an intrinsic mechanical attribute of the raw material. As a result of prior thermomechanical processing, the resultant strength attribute can be assigned primarily to the underlying microstructure that comprises the raw material. The causal relationship between material structure, in this instance, grain size, and the measurable strength, in this instance yield strength, is explained by the classical Hall-Petch relationship where strength is inversely proportional the square of grain size as given by,

$$\sigma_y \propto \frac{1}{\sqrt{G.S.}} \qquad\qquad (1)$$

wherein $\sigma_y$ is the yield strength as measured in MPa and G.S. is grain size as measured in millimeters as the average granular diameter. The strength attribute specifically affects the ability of the intravascular device to maintain vessel patency under *in-vivo* loading conditions.

The causal relationship between balloon-expandable device recoil (i.e. elastic "spring-back" upon initial unloading by deflation of the deployment catheter's balloon) and strength, in this instance yield strength, is principally affected by grain size. As previously described, a decrement in grain-size results in higher yield strength as shown above. Accordingly, the measurable device recoil is inversely proportional to the grain size of the material.

The causal relationship between fatigue resistance, in this instance endurance limit or the maximum stress below which a material can presumably endure an infinite number of stress cycles, and strength, in this instance yield strength, is principally affected by grain size. Although fatigue resistance is also affected by extrinsic factors such as existing material defects, for example, stable cracks and processing flaws, the principal intrinsic factor affecting fatigue resistance for a given applied load is material strength. As previously described, a decrement in grain-size results in higher yield strength as shown above. Accordingly, the endurance limit (i.e. fatigue resistance) is inversely proportional to the grain size of the material.

The causal relationship between ductility, in this instance the material's ability to support tensile elongation without observable material fracture (i.e. percent elongation), is significantly affected by grain size. Typically, ductility is inversely proportional to strength that would imply a direct relationship to grain size.

Microstructural attributes, in this instance, grain-size, may be configured to be equal to or less than about 32 microns in average diameter. In order to ensure that all of the measurable mechanical attributes are homogenous and isotropic within the intended structure or stent, an equiaxed distribution of granularity is preferable. So as to ensure that the structural properties of the intended stent are configured in the preferred manner, a minimum of about two structurally finite intergranular elements (i.e. grains) to a maximum of about ten structurally finite intergranular elements shall exist within a given region of the stent components or elements. In particular, the number of grains may be measured as the distance between the abluminal and the luminal surface of the stent component (i.e. wall thickness). While these microstructural aspects may be tailored throughout the entirety of the stent, it may be particularly advantageous to configure the deformable regions of the stent with these microstructural aspects as described in detail below.

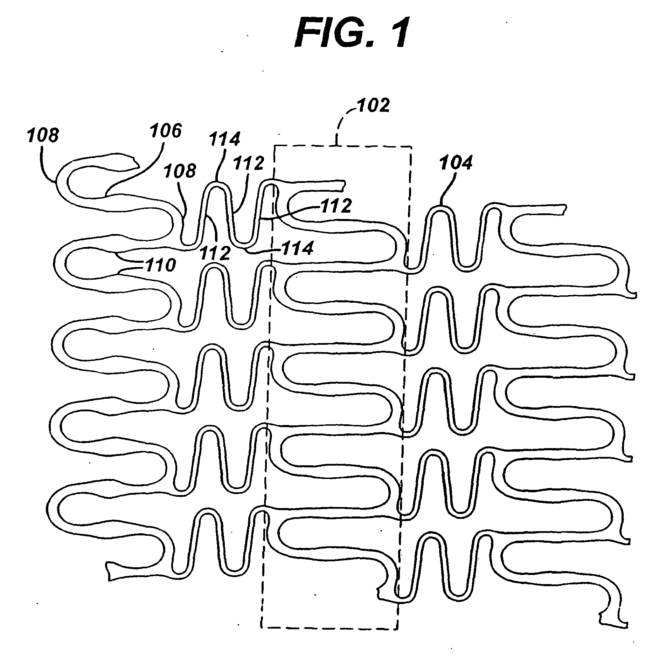

Referring to Figure 1, there is illustrated a partial planar view of a stent 100. The stent 100 comprises a plurality of hoop components 102 interconnected by a plurality of flexible connectors 104. The hoop components 102 are formed as a continuous series of substantially circumferentially oriented radial strut members 106 and alternating radial arc members 108. Although shown in planar view, the hoop components 102 are essentially ring members that are linked together by the flexible connectors 104 to form a substantially tubular stent structure. The combination of radial strut members 106 and alternating radial arc members 108 form a substantially sinusoidal pattern. Although the hoop components 102 may be designed with any number of design features and assume any number of configurations, in this example, the radial strut members 106 are wider in their central regions 110. This design feature may be utilized for a number of purposes, including, increased surface area for drug delivery.

The flexible connectors 104 are formed from a continuous series of substantially longitudinally oriented flexible strut members 112 and alternating flexible arc members 114. The flexible connectors 104, as described above, connect adjacent hoop components 102 together. In this example, the flexible connectors 104 have a substantially N-shape with one end being connected to a radial arc member on one hoop component and the other end being connected to a radial arc member on an adjacent hoop component. As with the hoop components 102, the flexible connectors 104 may comprise any number of design features and any number of configurations. In this example, the ends of the flexible connectors 104 are connected to different portions of the radial arc members of adjacent hoop components for ease of nesting during crimping of the stent. It is interesting to note that with this exemplary configuration, the radial arcs on adjacent hoop components are slightly out of phase, while the radial arcs on every other hoop component are substantially in phase. In addition, it is important to note that not every radial arc on each hoop component need be connected to every radial arc on the adjacent hoop component.

The substantially tubular structure of the stent 100 provides the scaffolding for maintaining the patentcy of substantially tubular organs, such as arteries. The stent 100 comprises a luminal surface and an abluminal surface. The distance between the two surfaces defines the wall thickness as is described in detail above. The stent 100 has an unexpanded diameter for delivery and an expanded diameter which roughly corresponds to the normal diameter of the organ into which it is delivered. As tubular organs such as arteries may vary in diameter, different size stents having different sets of unexpanded and expanded diameters may be designed without departing from the spirit of the present invention. As described herein, the stent 100 may be formed form any number of metallic materials, including cobalt-based alloys, iron-based alloys, titanium-based alloys, refractory-based alloys and refractory metals.

In the stent described above, a number of examples may be utilized to illustrate the relationship of equiaxed granularity to wall thickness. In the first example, the wall thickness may be varied in the range from about 0.013 mm (0.0005 inches) to about 0.152 mm (0.006 inches) for a stent having an expanded inside diameter of less than about 2.5 millimeters. Accordingly, for a maximal number of equiaxed grains, which in the exemplary embodiment is substantially not more than ten (10) discrete grains across the thickness of the wall, the equiaxed grain size shall be equal to or greater than substantially 1.25 microns. This dimensional attribute may be arrived at by simply dividing the minimal available wall thickness by the maximal number of available equiaxed grains. In another example, the wall thickness may be varied in the range from about 0.051 mm (0.002 inches) to about 0.203 mm (0.008 inches) for a stent having an expanded inside diameter from about 2.5 millimeters to about 5.0 millimeters. Accordingly, for a maximal number of equiaxed grains, which in the exemplary embodiment is substantially not more than ten (10) discrete grains across the thickness of the wall, the equiaxed grain size shall be equal to or greater than substantially 5.0 microns. In yet another example, the wall thickness may be varied in the range from about 0.102 mm (0.004 inches) to about 0.305 mm (0.012 inches) for a stent having an expanded inside diameter from about 5.0 millimeters to about 12.0 millimeters. Accordingly, for a maximal number of equiaxed grains, which in the exemplary embodiment is substantially not more than ten (10) discrete grains across the thickness of the wall, the equiaxed grain size shall be equal to or greater than substantially 10.0 microns. In yet still another example, the wall thickness may be varied in the range from about 0.152 mm (0.006 inches) to about 0.635 mm (0.025 inches) for a stent having an expanded inside diameter from about 12.0 millimeters to about 50.0 millimeters. Accordingly, for a maximal number of equiaxed grains, which in the exemplary embodiment is substantially not more than ten (10) discrete grains across the thickness of the wall, the equiaxed grain size shall be equal to or greater than substantially 15.0 microns. In making the above calculations, it is important to maintain rigorous consistency of dimensional units.

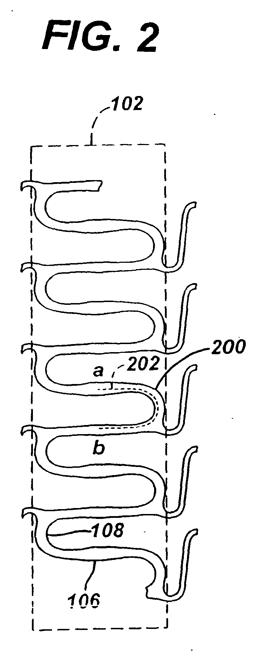

The elements of the stent 100, illustrated in Figure 1, may be further defined in terms that may be utilized to describe the relationship between geometry, material and the effects of applied loading. Referring to Figure 2, there is illustrated, in planar view, a single hoop component 102. As described above, the hoop component 102 is formed as a series of substantially circumferentially oriented radial strut members 106 and alternating radial arc members 108. However, the hoop component 102 may also be defined as a number of interconnected loops, wherein a single loop is the element between point a and point b in Figure 2. In other words, each single loop comprises a portion of two radial strut members and an entire radial arc member. Formulaically, the linear length of a single loop, $L_L$, may be given by

$$L_L = RS_L + RA_L, \qquad\qquad\qquad (2)$$

wherein $RS_L$ is the length of a strut member and $RA_L$ is the linear length of the arc member as measured through its center line. Given that the hoop 102 may be defined as a number of interconnected loops, the total linear path length of a hoop, $H_L$, may be given by

$$H_L = \Sigma\, L_L. \qquad\qquad\qquad (3)$$

From the expressions represented by equations (2) and (3) a number of ratios may be developed that describe or define the relationship between geometry, material and the effects of applied load. More specifically, it is the unique material composition and built in properties, i.e. microstructure, that provide the means for fabricating a stent with various geometries that are able to withstand the various loading conditions as is described in detail subsequently. For example, a stent may be designed such that each radial strut's member is configured to exhibit substantially no permanent plastic deformation upon expansion while each radial arc member is configured to accommodate substantially all permanent plastic deformation upon expansion. Alternately, a stent may be designed such that each radial arc member is configured to exhibit substantially no permanent plastic deformation upon expansion, while each radial strut member is configured to accommodate substantially all permanent deformation upon expansion. As these two examples represent the two extremes, it is important to note that the present invention also applies to the continuum between these extremes.

The material properties that are of importance relate to the microstructure as described in detail above. Specifically, the stents are fabricated from a metallic material processed to have a microstructure with a granularity of about thirty-two microns or less and comprise from about two to about ten substantially equiaxed grains as measured across the wall thickness of the stent. The ratios set forth below help describe the desirable properties of the stent.

The expansion efficiency ratio, $H_{eff}$, is given by

$$H_{eff} = C/H_L, \qquad\qquad\qquad (4)$$

wherein C is the circumference of a fully expanded hoop (or stent) and $H_L$ is the total path length of a hoop as set forth in equation (3). Due to the metallic materials and associated built-in properties thereof, the ratio of equation (4) that may be achieved is given by

$$H_{eff} = C/H_L > 0.25. \qquad\qquad\qquad (5)$$

In other words, the ratio of the circumference of a fully expanded hoop to the total path of the hoop is greater than 0.25. Obviously, the maximum that this ratio may achieve is unity since the path length should not be greater than the circumference of the expanded hoop. However, it is this 0.25 expansion efficiency ratio that is important. In any stent design it is desirable to minimize the amount of structural metal within the vessel and to reduce the overall complexity of fabrication. Expansion efficiency ratios of greater than 0.25 are achievable through the utilization of these new materials. It is important to note that the circumference of a fully expanded hoop should substantially correspond to the normal luminal circumference of the vessel into which the stent is placed. In addition, if the lumen of the vessel is not substantially circular, perimeter may be substituted for circumference, C.

The loop efficiency ratio, $L_{eff}$, is given by

$$L_{eff} = L_L / RA_L, \qquad\qquad (6)$$

wherein $L_L$ is the linear length or path-length of a single loop given by equation (2) and $RA_L$ is the linear length or path-length of an arc member. Using the elementary rules of algebraic substitution while maintaining rigorous dimensional integrity, Equation (6) may be rewritten as

$$L_{eff} = (RS_L + RA_L) / RA_L \qquad\qquad (7)$$

.

As may be easily seen from Equation (7), the loop efficiency ratio may never be less than unity. However, because of the material properties, the linear length or path-length of the arc and the linear length or path-length of the struts may be manipulated to achieve the desired characteristics of the final product. For example, under the condition where the strain is primarily carried within the radial arc member, increasing the length of the radial strut for a fixed expansion diameter (displacement controlled phenomena) reduces the magnitude of the non-recoverable plastic strain integrated across the entirety of the radial arc. Similarly, under the condition where the strain is primarily carried within the radial strut member, increasing the length of the radial strut for a fixed expansion diameter (displacement controlled phenomena) reduces the magnitude of the non-recoverable plastic strain integrated across the entirety of the radial strut. In addition, under the condition where the strain is primarily carried within the radial arc member, increasing the path-length of the radial arc for a fixed expansion diameter (displacement controlled phenomena) reduces the magnitude of the non-recoverable plastic strain integrated across the entirety of the radial arc. As these examples represent the extremes, it is important to note that the present invention also applies to the continuum between these extremes.

Accordingly, since the material is able to withstand greater loading, various designs based upon the above ratios may be achieved.

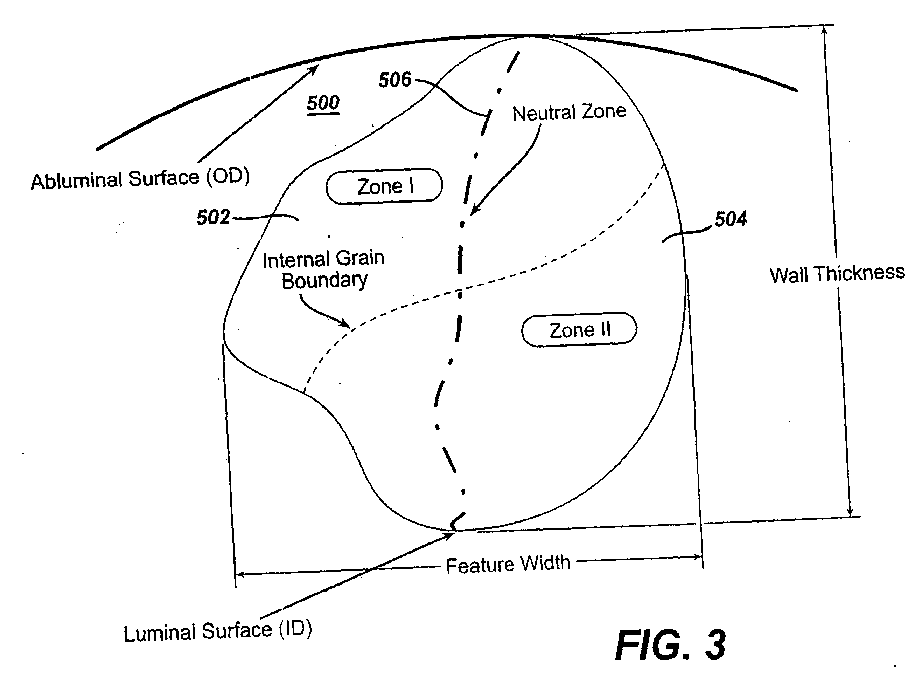

It is important to note that no assumption is made as to the symmetry of the radial struts or radial arc that comprise each single loop and the hoops of the structure. Furthermore, these principals also apply to loops that are interconnected along the longitudinal axis but not necessarily along the radial axis, for example, loops configured into a helical structure. Although a single loop has been illustrated with a single arc member, it obvious to those of ordinary skill in the art, a single loop may be comprise no radial arcs, a single radial arc (as illustrated in Figures 1 and 2) and/or multiple radial arcs and no radial strut, a single radial strut and/or multiple radial struts (as illustrated in Figure 3 and 4).

Intraluminal scaffolds or stents may comprise any number of design configurations and materials depending upon the particular application and the desired characteristics. One common element of all stent designs is that each stent comprises at least one load-bearing element. Typically, the load-bearing elements have well defined geometries; however, alternate non-conventional geometries may be described in-terms of a bounded cross-sectional area. These bounded areas may be engineered to have either an asymmetric or symmetric configuration. Regardless of the configuration, any bounded cross-sectional area should include at least one internal grain boundary. Those skilled in the art will recognize that the grain-boundary identified in this exemplary embodiment should preferably not constitute any measurable degree of the surface defined by the perimeter of the bounded cross-sectional area. Additionally, those skilled in the art will understand that the grain-boundary discussed in this exemplary embodiment should preferably be characterized as having a high-angle (i.e. typically greater than or equal to about 35 degrees) crystallographic interface. Also, in the presence of microstructural defects such as microcracks (i.e. lattice level discontinuities that can be characterized as planar crystallographic defects), the fatigue crack growth-rate will be expected to be proportional to the number of grains that exist within the bounded cross-sectional area. Since there is one internal grain boundary, this ensures that at least two discrete grains or portions thereof will exist within the bounded cross-sectional area. As described herein, the well-known Hall-Petch relationship that inversely relates grain-size to strength should be observed in this exemplary embodiment as the average grain-size will proportionally decrease as the number of grains within the bounded cross-sectional area increases. In addition, as the number of grains increase within the bounded cross-sectional area, the ability for the microstructure to internally accommodate stress-driven grain boundary sliding events will also increase and should preferably increase localized ductility.

Referring to Figure 3, there is illustrated a cross-sectional representation of a load-bearing stent element 500. As shown, the bounded cross-sectional area comprises a first zone 502, a second zone 504 and a neutral zone 506 which are the result of a stress gradient that is directly proportional to the external loading conditions. The neutral zone 506 is generally defined as a substantially stress free zone that exists between and is bounded by the first zone 502 and the second zone 504. As a function of changing external loading conditions either from the unloaded condition or a loaded condition, the first and second zones, 502 and 504, will undergo a change in tensile and/or compressive stress. It is important to note that the zone assignments shown in Figure 5 are illustrative in nature and not intended to define relative positioning within the bounded area. The load bearing stent element 500 has a wall thickness that is defined as the radial distance between the luminal surface and the abluminal surface. The load bearing element 500 also has a feature width. The feature width is defined as the linear distance across the first zone 502, neutral zone 506 and the second zone 504 in a direction that is substantially orthogonal to the wall thickness. It is important to note that the feature width is measured at a point that represents the greatest measurable distance in a direction that is substantially orthogonal to the wall thickness.

The exemplary load-bearing stent element 500 that is illustrated in Figure 3 may be fabricated from a substantially polymeric material system. The properties and attributes described above, that are recognizable by one of appropriate skill and technical qualification in the relevant art, may be utilized to produce a load-bearing structure that is substantially similar to that created with the metallic materials described above.

According to the present invention, an intraluminal scaffold element may be fabricated from a non-metallic material such as a polymeric material including non-crosslinked thermoplastics, cross-linked thermosets, composites and blends thereof. There are typically three different forms in which a polymer may display the mechanical properties associated with solids; namely, as a crystalline structure, as a semi-crystalline structure and/or as an amorphous structure. All polymers are not able to fully crystallize, as a high degree of molecular regularity within the polymer chains is essential for crystallization to occur. Even in polymers that do substantially crystallize, the degree of crystallinity is generally less than 100 percent. Within the continuum between fully crystalline and amorphous structures, there are two thermal transitions possible; namely, the crystal-liquid transition (i.e. melting point temperature, $T_m$) and the glass-liquid transition (i.e. glass transition temperature, $T_g$). In the temperature range between these two transitions there may be a mixture of orderly arranged crystals and chaotic amorphous polymer domains.

The Hoffman-Lauritzen theory of the formation of polymer crystals with "folded" chains owes its origin to the discovery in 1957 that thin single crystals of polyethylene may be grown from dilute solutions. Folded chains are preferably required to form a substantially crystalline structure. Hoffman and Lauritzen established the foundation of the kinetic theory of polymer crystallization from "solution" and "melt" with particular attention to the thermodynamics associated with the formation of chain-folded nuclei.

Crystallization from dilute solutions is required to produce single crystals with macroscopic perfection (typically magnifications in the range of about 200x to about 400x). Polymers are not substantially different from low molecular weight compounds such as inorganic salts in this regard. Crystallization conditions such as temperature, solvent and solute concentration may influence crystal formation and final form. Polymers crystallize in the form of thin plates or "lamellae." The thickness of these lamellae is on the order of 10 nanometers (i.e. nm). The dimensions of the crystal plates perpendicular to the small dimensions depend on the conditions of the crystallization but are many times larger than the thickness of the platelets for a well-developed crystal. The chain direction within the crystal is along the short dimension of the crystal, which indicates that, the molecule folds back and forth (e.g. like a folded fire hose) with successive layers of folded molecules resulting in the lateral growth of the platelets. A crystal does not consist of a single molecule nor does a molecule reside exclusively in a single crystal. The loop formed by the chain as it emerges from the crystal turns around and reenters the crystal. The portion linking the two crystalline sections may be considered amorphous polymer. In addition, polymer chain ends disrupt the orderly fold patterns of the crystal, as described above, and tend to be excluded from the crystal. Accordingly, the polymer chain ends become the amorphous portion of the polymer. Therefore, no currently known polymeric material can be 100 percent crystalline. Post polymerization processing conditions dictate the crystal structure to a substantial extent.

Single crystals are not observed in crystallization from bulk processing. Bulk crystallized polymers from melt exhibits domains called "spherulites" that are symmetrical around a center of nucleation. The symmetry is perfectly circular if the development of the spherulite is not impinged by contact with another expanding spherulite. Chain folding is an essential feature of the crystallization of polymers from the molten state. Spherulites are composed of aggregates of "lamellar" crystals radiating from a nucleating site. Accordingly, there is a relationship between solution and bulk grown crystals.

The spherical symmetry develops with time. Fibrous or lathlike crystals begin branching and fanning out as in dendritic growth. As the lamellae spread out dimensionally from the nucleus, branching of the crystallites continue to generate the spherical morphology. Growth is accomplished by the addition of successive layers of chains to the ends of the radiating laths. The chain structure of polymer molecules suggests that a given molecule may become involved in more than one lamella and thus link radiating crystallites from the same or adjacent spherulites. These interlamellar links are not possible in spherulites of low molecular weight compounds, which show poorer mechanical strength as a consequence.

The molecular chain folding is the origin of the "Maltese" cross, which identifies the spherulite under crossed polarizers. For a given polymer system, the crystal size distribution is influenced by the initial nucleation density, the nucleation rate, the rate of crystal growth, and the state of orientation. When the polymer is subjected to conditions in which nucleation predominates over radial growth, smaller crystals result. Larger crystals will form when there are relatively fewer nucleation sites and faster growth rates. The diameters of the spherulites may range from about a few microns to about a few hundred microns depending on the polymer system and the crystallization conditions.

Therefore, spherulite morphology in a bulk-crystallized polymer involves ordering at different levels of organization; namely, individual molecules folded into crystallites that in turn are oriented into spherical aggregates. Spherulites have been observed in organic and inorganic systems of synthetic, biological, and geological origin including moon rocks and are therefore not unique to polymers.

Stress induced crystallinity is important in film and fiber technology. When dilute solutions of polymers are stirred rapidly, unusual structures develop which are described as having "shish kebab" morphology. These consist of chunks of folded chain crystals strung out along a fibrous central column. In both the "shish" and the "kebab" portions of the structure, the polymer chains are parallel to the overall axis of the structure.

When a polymer melt is sheared and quenched to a thermally stable condition, the polymer chains are perturbed from their random coils to easily elongate parallel to the shear direction. This may lead to the formation of small crystal aggregates from deformed spherulites. Other morphological changes may occur, including spherulite to fibril transformation, polymorphic crystal formation change, reorientation of already formed crystalline lamellae, formation of oriented crystallites, orientation of amorphous polymer chains and/or combinations thereof.

It is important to note that polymeric materials may be broadly classified as synthetic, natural and/or blends thereof. Within these broad classes, the materials may be defined as biostable or biodegradable. Examples of biostable polymers include polyolefins, polyamides, polyesters, fluoropolymers, and acrylics. Examples of natural polymers include polysaccharides and proteins. Examples of biodegradable polymers include the family of polyesters such as polylactic acid, polyglycolic acid, polycaprolactone, polytrimethylene carbonate and polydioxanone. Additional examples of biodegradable polymers include polyhydroxalkanoates such as polyhydroxybutyrate-co-valerates; polyanhydrides; polyorthoesters; polyaminoacids; polyesteramides; polyphosphoesters; and polyphosphazenes. Copolymers and blends of any of the described polymeric materials may be utilized in accordance with the present invention.

When constructing an intraluminal stent from polymeric materials, a maximum spherulitic size of about 50 microns or less was necessary to achieve the functional properties and attributes described herein.

## Claims

1. An intraluminal scaffold (500) comprising at least one load bearing element having a luminal surface and an abluminal surface, the load bearing element having a predetermined wall thickness, wherein the wall thickness is defined by the radial distance between the luminal surface and the abluminal surface, and a predetermined feature width, wherein an area bounded by the wall thickness and the feature width comprises three zones, a first zone (502), a second zone (504) and a neutral zone (506), which are the result of a stress gradient that is directly proportional to external loading conditions, and wherein when there is a change in external loading conditions either from a loaded or an unloaded condition, the first zone (502) undergoes a change in compressive and/or tensile stress due to the change in external load, the second zone (504) undergoes a change in tensile and/or compressive stress due to change in the external load and the neutral zone (506) is substantially stress free and is between the first and second zones, the feature width being the linear distance across the first, neutral and second zones in a direction substantially orthogonal to the wall thickness, the load bearing element being fabricated from a non-metallic material processed to have a microstructure with structural domains having a size of 50 microns or less and at least one internal structural boundary within the bounded area.

2. The intraluminal scaffold according to claim 1, wherein the material is formed from a synthetic polymeric material.

3. The intraluminal scaffold according to claim 2, wherein the synthetic polymeric material comprises polyolefins.

4. The intraluminal scaffold according to claim 2, wherein the synthetic polymeric material comprises polyamides.

5. The intraluminal scaffold according to claim 2, wherein the synthetic polymeric material comprises polyesters.

6. The intraluminal scaffold according to claim 2, wherein the synthetic polymeric material comprises fluoropolymers.

7. The intraluminal scaffold according to claim 1, wherein the material is formed from a natural polymeric material.

**8.** The intraluminal scaffold according to claim 7, wherein the natural polymeric material comprises polysacaccharides.

**9.** The intraluminal scaffold according to claim 7, wherein the natural polymeric material comprises proteins.

**10.** The intraluminal scaffold according to claim 1, wherein the material is formed from a synthetic biodegradable polymeric material.

**11.** The intraluminal scaffold according to claim 10, wherein the biodegradable polymeric material comprises polyesters.

**12.** The intraluminal scaffold according to claim 10, wherein the biodegradable polymeric material comprises polyhydroxalkanoates.

**13.** The intraluminal scaffold according to claim 10, wherein the biodegradable polymeric material comprises polyanhydrides.

**14.** The intraluminal scaffold according to claim 10, wherein the biodegradable polymeric material comprises polyorthoesters.

**15.** The intraluminal scaffold according to claim 10, wherein the biodegradable polymeric material comprises polyaminoacids.

**16.** The intraluminal scaffold according to claim 10, wherein the biodegradable polymeric material comprises polyesteramides.

**17.** The intraluminal scaffold according to claim 10, wherein the biodegradable polymeric material comprises polyphosphoesters.

**18.** The intraluminal scaffold according to claim 10, wherein the biodegradable polymeric material comprises polyphosphazenes.

**19.** The intraluminal scaffold according to claim 1, wherein the domains comprise spherulitic structures.

**20.** The intraluminal scaffold according to claim 1, wherein the domains comprise folded-chain structures.

**21.** The intraluminal scaffold according to claim 1, wherein the domains comprise spherulitic and folded-chain structures.

**Patentansprüche**

**1.** Intraluminales Gerüst (500), das wenigstens ein tragendes Element umfasst mit einer luminalen Oberfläche und einer abluminalen Oberfläche, wobei das tragende Element eine vorbestimmte Wandstärke aufweist, wobei die Wandstärke definiert ist durch den radialen Abstand zwischen der luminalen Oberfläche und der abluminalen Oberfläche, und eine vorbestimmte Eigenschaftsbreite, wobei ein Bereich, der durch die Wandstärke und die Eigenschaftsbreite begrenzt ist, drei Zonen aufweist, eine erste Zone (502), eine zweite Zone (504) und eine neutrale Zone (506), die das Ergebnis eines Spannungsgradienten sind, der direkt proportional zu externen Belastungszuständen ist, und wobei beim Vorliegen einer Änderung der externen Belastungszustände aus entweder einem belasteten oder einem unbelasteten Zustand die erste Zone (502) eine Änderung der Druckspannung und/oder der Zugspannung aufgrund der Änderung der externen Belastung durchmacht, die zweite Zone (504) eine Änderung in der Zugspannung und/oder der Druckspannung aufgrund der Änderung der externen Belastung durchmacht und die neutrale Zone (506) im Wesentlichen spannungsfrei ist und sich zwischen der ersten und der zweiten Zone befindet, wobei die Eigenschaftsbreite die lineare Entfernung über die erste, die neutrale und die zweite Zone in der Richtung im Wesentlichen orthogonal zu der Wandstärke ist, das tragende Element aus einem nicht metallischen Material hergestellt ist, welches so bearbeitet ist, dass es eine Mikrostruktur aufweist mit strukturellen Domänen, welche eine Größe von 50 μm oder weniger besitzen und wenigstens eine interne strukturelle Begrenzung innerhalb des begrenzten Bereichs.

**2.** Intraluminales Gerüst nach Anspruch 1, bei welchem das Material aus einem synthetischen Polymermaterial gebildet ist.

**3.** Intraluminales Gerüst nach Anspruch 2, bei welchem das synthetische Polymermaterial Polyolefine umfasst.

**4.** Intraluminales Gerüst nach Anspruch 2, bei welchem das synthetische Polymermaterial Polyamide umfasst.

**5.** Intraluminales Gerüst nach Anspruch 2, bei welchem das synthetische Polymermaterial Polyester umfasst.

**6.** Intraluminales Gerüst nach Anspruch 2, bei welchem das synthetische Polymermaterial Fluoropolymere umfasst.

**7.** Intraluminales Gerüst nach Anspruch 1, bei welchem das Material aus einem natürlichen Polymermaterial gebildet ist.

**8.** Intraluminales Gerüst nach Anspruch 7, bei welchem das natürliche Polymermaterial Polysaccharide umfasst.

**9.** Intraluminales Gerüst nach Anspruch 7, bei welchem das natürliche Polymermaterial Proteine umfasst.

**10.** Intraluminales Gerüst nach Anspruch 1, bei welchem das Material aus einem synthetischen biologisch abbaubaren Polymermaterial gebildet ist.

**11.** Intraluminales Gerüst nach Anspruch 10, bei welchem das biologisch abbaubare Polymermaterial Polyester umfasst.

**12.** Intraluminales Gerüst nach Anspruch 10, bei welchem das biologisch abbaubare Polymermaterial Polyhydroxalkanoate umfasst.

**13.** Intraluminales Gerüst nach Anspruch 10, bei welchem das biologisch abbaubare Polymermaterial Polyanhydride umfasst.

**14.** Intraluminales Gerüst nach Anspruch 10, bei welchem das biologisch abbaubare Polymermaterial Polyorthoester umfasst.

**15.** Intraluminales Gerüst nach Anspruch 10, bei welchem das biologisch abbaubare Polymermaterial Polyaminosäuren umfasst.

**16.** Intraluminales Gerüst nach Anspruch 10, bei welchem das biologisch abbaubare Polymermaterial Polyesteramide umfasst.

**17.** Intraluminales Gerüst nach Anspruch 10, bei welchem das biologisch abbaubare Polymermaterial Polyphosphoester umfasst.

**18.** Intraluminales Gerüst nach Anspruch 10, bei welchem das biologisch abbaubare Polymermaterial Polyphosphazene umfasst.

**19.** Intraluminales Gerüst nach Anspruch 1, bei welchem die Domänen sphärolitische Strukturen umfasst.

**20.** Intraluminales Gerüst nach Anspruch 1, bei welchem die Domänen Strukturen mit gefalteten Ketten umfasst.

**21.** Intraluminales Gerüst nach Anspruch 1, bei welchem die Domänen sphärolitische Strukturen und Strukturen mit gefalteten Ketten umfasst.


**Revendications**

**1.** Tuteur intraluminal (500) comprenant au moins un élément porteur de charge ayant une surface luminale et une surface abluminale, l'élément porteur de charge ayant une épaisseur de paroi prédéterminée, dans lequel l'épaisseur de paroi est définie par la distance radiale entre la surface luminale et la surface abluminale, et une largeur caractéristique prédéterminée, dans lequel une zone limitée par l'épaisseur de paroi et la largeur caractéristique comprend trois zones, une première zone (502), une seconde zone (504) et une zone neutre (506), qui sont le résultat d'un gradient de contrainte qui est directement proportionnel aux conditions de charge extérieures et dans lequel, lorsqu'un changement des conditions de charge externes se produit, que ce soit d'une condition chargée ou déchargée, la première zone (502) subit un changement de contrainte par compression et/ou traction du fait du changement de charge externe, la seconde zone (504) subit un changement de contrainte de traction et/ou de compression du fait du changement de la charge externe et la zone neutre (506) est sensiblement sans contrainte et se trouve entre la première et la seconde zones, la largeur caractéristique étant la distance linéaire allant de la première zone, la zone neutre à la seconde zone, dans une direction sensiblement perpendiculaire à l'épaisseur de paroi, l'élément porteur de charge étant fabriqué à partir d'un matériau non métallique traité de façon à obtenir une microstructure avec des domaines structurels ayant une taille de 50 microns ou moins et au moins une limite structurelle interne dans la zone limitée.

**2.** Tuteur intraluminal selon la revendication 1, dans lequel le matériau est formé à partir d'un matériau polymère synthétique.

**3.** Tuteur intraluminal selon la revendication 2, dans lequel le matériau polymère synthétique comprend des polyoléfines.

**4.** Tuteur intraluminal selon la revendication 2, dans lequel le matériau polymère synthétique comprend des polyamides.

**5.** Tuteur intraluminal selon la revendication 2, dans lequel le matériau polymère synthétique comprend des polyesters.

**6.** Tuteur intraluminal selon la revendication 2, dans lequel le matériau polymère synthétique comprend des fluoropolymères.

**7.** Tuteur intraluminal selon la revendication 1, dans lequel le matériau est formé d'un matériau polymère naturel.

**8.** Tuteur intraluminal selon la revendication 7, dans lequel le matériau polymère naturel comprend des polysaccharides.

**9.** Tuteur intraluminal selon la revendication 7, dans lequel le matériau polymère naturel comprend des protéines.

**10.** Tuteur intraluminal selon la revendication 1, dans lequel le matériau est formé à partir d'un matériau polymère biodégradable synthétique.

**11.** Tuteur intraluminal selon la revendication 10, dans lequel le matériau polymère biodégradable comprend des polyesters.

**12.** Tuteur intraluminal selon la revendication 10, dans lequel le matériau polymère biodégradable comprend des polyhydroxyalcanoates.

**13.** Tuteur intraluminal selon la revendication 10, dans lequel le matériau polymère biodégradable comprend des polyanhydrides.

**14.** Tuteur intraluminal selon la revendication 10, dans lequel le matériau polymère biodégradable comprend des polyorthoesters.

**15.** Tuteur intraluminal selon la revendication 10, dans lequel le matériau polymère biodégradable comprend des polyaminoacides.

**16.** Tuteur intraluminal selon la revendication 10, dans lequel le matériau polymère biodégradable comprend des polyesteramides.

**17.** Tuteur intraluminal selon la revendication 10, dans lequel le matériau polymère biodégradable comprend des polyphosphoesters.

**18.** Tuteur intraluminal selon la revendication 10, dans lequel le matériau polymère biodégradable comprend des polyphosphazènes.

**19.** Tuteur intraluminal selon la revendication 1, dans lequel les domaines comprennent des structures sphérolitiques.

**20.** Tuteur intraluminal selon la revendication 1, dans lequel les domaines comprennent des structures en chaîne repliée.

21. Tuteur intraluminal selon la revendication 1, dans lequel les domaines comprennent des structures sphérolitiques et en chaîne repliée.

## FIG. 1

# FIG. 2

Abluminal Surface (OD)

**500**

506

Neutral Zone

Zone I

502

Internal Grain
Boundary

504

Zone II

Wall Thickness

Feature Width

Luminal Surface (ID)

*FIG. 3*

EP 1 645 300 B1

**EP 1 645 300 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 0083732 A